(19) Europäisches Patentamt
European Patent Office
Office européen des brevets

(11) **EP 2 404 636 B1**

(12) **EUROPEAN PATENT SPECIFICATION**

(45) Date of publication and mention of the grant of the patent:
**07.05.2014 Bulletin 2014/19**

(21) Application number: **10748737.3**

(22) Date of filing: **02.03.2010**

(51) Int Cl.:
**B21C 1/00** *(2006.01)*   **B21C 37/04** *(2006.01)*
**B21J 9/06** *(2006.01)*   **C21D 7/10** *(2006.01)*
**C21D 8/06** *(2006.01)*   **A61M 25/09** *(2006.01)*
**C21D 1/00** *(2006.01)*   **C21D 7/00** *(2006.01)*

(86) International application number:
**PCT/JP2010/053324**

(87) International publication number:
**WO 2010/101142 (10.09.2010 Gazette 2010/36)**

(54) **CORE FOR GUIDE WIRE AND METHOD OF PRODUCING SAME**

KERN FÜR EINEN FÜHRUNGSDRAHT UND VERFAHREN ZU SEINER HERSTELLUNG

AME POUR FIL-GUIDE ET SON PROCÉDÉ DE FABRICATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.03.2009 JP 2009051274**

(43) Date of publication of application:
**11.01.2012 Bulletin 2012/02**

(73) Proprietor: **Piolax Medical Devices, Inc.**
**Yokohama-shi**
**Kanagawa 240-0023 (JP)**

(72) Inventors:
• **YAMAUCHI Kiyoshi**
  **Sendai-shi**
  **Miyagi 980-0801 (JP)**
• **FURUKAWA Akihisa**
  **Sendai-shi**
  **Miyagi 980-8577 (JP)**
• **ISHIDA Kiyohito**
  **Sendai-shi**
  **Miyagi 980-8577 (JP)**
• **ISHIKAWA Takeshi**
  **Yokohama-shi**
  **Kanagawa 240-0023 (JP)**
• **TAKEZAWA Kiyonori**
  **Fukui-shi**
  **Fukui 919-0321 (JP)**
• **TAKEZAWA Mitsuya**
  **Fukui-shi**
  **Fukui 919-0321 (JP)**

(74) Representative: **Grünecker, Kinkeldey, Stockmair & Schwanhäusser**
**Leopoldstrasse 4**
**80802 München (DE)**

(56) References cited:
WO-A2-01/41859       GB-A- 2 347 685
JP-A- 5 293 175       JP-A- 11 090 584
JP-U- 7 007 653       US-A- 1 635 793
US-A1- 2003 100 847   US-A1- 2008 281 396

EP 2 404 636 B1

## Description

## Technical Field

The present invention relates to a core wire for guide wire which serves as a material of a guide wire for use in introduction of a catheter etc., and a method for manufacturing the core wire.

## Background Art

In the background art, contrast media or medical agents are dosed through a catheter for inspection or treatment in a tubular organ such as a blood vessel or a trachea. The catheter is inserted to an intended place through a narrow and soft guide wire. For radiography or treatment in a coronary artery, a cerebral artery or another narrow tubular organ whose diameter is reduced due to disease, a microscopic catheter is used and a microscopic guide wire is also used correspondingly.

The guide wire is required to have properties such as pushability with which a pushing force on the operating side can be transmitted to a front end portion of the guide wire or torque transmission characteristic with which a blood vessel can be selected in a bifurcated vessel.

Stainless steel, Ti-Ni based alloy, etc. may be used as the material of such a guide wire.

The stainless steel is hard and has high rigidity. Therefore, a narrow-diameter guide wire formed of the stainless steel is superior in pushability. However, since the stainless steel is brought into fatigue deformation easily, the original performance and function of the stainless steel guide wire is apt to be spoiled in the course of use.

On the other hand, the Ti-Ni based alloy is superior in flexibility so as to be prevented from fatigue deformation. However, since the Ti-Ni based alloy is softer and lower in rigidity than stainless steel, the microscopic guide wire formed of the Ti-Ni based alloy is apt to be insufficient in pushability, torque transmission characteristic, etc. Thus, therehavebeen some proposals for guide wires made of Ti-Ni based alloys with enhanced rigidity.

For example, Patent Document 1 discloses a catheter guide wire including a core material and a coating portion applied on the surface of the core material, wherein the core material is formed of a Ti-Ni based alloy containing 45 to 52 at% Ni, and at least one of an exothermic amount and an endothermic amount of the Ti-Ni based alloy caused by martensitic transformation and martensitic reverse transformation is not higher than 0.80 cal/g.

Patent Document 2 discloses catheter guide wire including a Ti-Ni alloy core wire and an outer circumferential member applied on the core wire from outside, wherein a sectional area of a front end portion of the core wire is smaller than that of any other portion of the core wire by a chemical process and/or a mechanical process. An example of the aforementioned mechanical process is swaging or rolling.

Patent Document 3 discloses a method for manufacturing a catheter guide wire in which a sectional area of a front end portion of a Ti-Ni alloy core wire having superelasticity in a temperature range of from 0°C to 40°C is made smaller than that of any other portion of the core wire by at least one process selected from a group consisting of etching processing, cutting/grinding and swaging, and the core wire obtained thus is coated with an outer circumferential member from outside.

## Prior Technical Documents

## Patent Documents

Patent Document 1: JP-H05-293175-A
Patent Document 2: JP-H06-165822-A
Patent Document 3: JP-H11-128363-A
Patent Document 4: WO-A-01/41859

## Summary of the Invention

## Problems that the Invention is to Solve

Since the guide wire of Patent Document 1 has a Young's modulus of about 40 GPa as shown in its test example, sufficient rigidity to be used for microscopic blood vessel may not be obtained.

In Patent Document 2, the front end portion of the core wire in the guide wire is processed to be narrower than any other portion of the core wire by a chemical process and/or a mechanical process. When the front end portion of the core wire is processed by a mechanical process, the rigidity of the front end portion of the core wire is enhanced by work hardening. However, an intermediate portion and a base end portion of the core wire are not processed at all but remain soft. The rigidity of the core wire as a whole is not enhanced. Thus, a pushing force acting at the base end portion of the core wire cannot be transmitted to the front end portion efficiently, and there is a problem in terms of pushability.

Also in Patent Document 3, only the front end portion of the core wire is processed by cutting, swaging etc. The rigidity in the intermediate portion and the base end portion of the core wire cannot be enhanced, and there is a problem in terms of pushability as in Patent Document 2.

An object of the present invention is to provide a core wire for guide wire in which high rigidity can be attained even with a fine wire diameter so that pushability is improved while the core wire is prevented from fatigue deformation, and a method for manufacturing the core wire.

**Means for Solving the Problems**

A first invention as described in the claims provides a core wire for guide wire, wherein the core wire is made of a Ti-Ni based alloy, has a wire diameter not larger than 0.5 mm and has a Young' s modulus not lower than 50 GPa.

According to the above invention, the core wire has high rigidity to be not lower than 50 GPa in Young's modulus while the core wire has the fine wire diameter not larger than 0.5 mm. Thus, the core wire has high pushability which is required when the core wire is inserted into a tubular organ, and the core wire is prevented from fatigue deformation. Accordingly, the core wire can be used suitably for guide wire, for example, to be inserted into a narrow blood vessel of a heart, a brain, etc.

A second invention provides, based on the first invention, the core wire, wherein wire drawing and swaging are performed on the core wire.

According to the above invention, the two processes of wire drawing and swaging are performed so that the working degree of the core wire is improved. Thus, high rigidity canbe attained in spite of the fine wire diameter.

A third invention provides, based on the first or second invention, the core wire, wherein the Ti-Ni based alloy is a work-hardened Ti-Ni based alloy whose texture has a microcrystalline structure.

In the case where either the wire drawing or the swaging alone is performed alone, the texture has a structure close to an amorphous structure. When both the wire drawing and the swaging are performed as in the core wire, the texture has a microcrystalline structure. As a result, prevention from fatigue deformation can be attained even in the processed state of the core wire while the mechanical characteristics are improved to keep the rigidity. Thus, the core wire can be used suitably for microscopic guide wire.

A fourth invention provides, based on the second or third invention, the core wire, wherein heat treatment as well as the wire drawing and the swaging is performed on the core wire.

According to the above invention, the heat treatment is performed so that strain or residual stress caused by the wire drawing and the swaging can be relaxed. Thus, the linearity which is essential as a core wire for guide wire can be enhanced while the rigidity is attained.

A fifth invention provides, based on any one of the first to fourth inventions, a method for manufacturing the core wire including: drawing a raw material made of a Ti-Ni based alloy, and then carrying out swaging to obtain a core wire having a wire diameter not larger than 0.5 mm and a Young's modulus not lower than 50 GPa.

According to the above invention, the two processes of wire drawing and swaging are performed. Thus, a core wire which has high rigidity to be not lower than 50 GPa in Young's modulus and which is prevented from fatigue deformation can be obtained.

A sixth invention provides, based on the fifth invention, the method further including: performing heat treatment after the swaging.

According to the above invention, strain or residual stress caused by the wire drawing and the swaging can be eliminated by the heat treatment. Thus, it is possible to obtain a core wire which has rigidity and is prevented from fatigue deformation and which has high linearity.

**Effect of the Invention**

According to the invention the core wire has high rigidity to be not lower than 50 GPa in Young's modulus while the core wire has a fine wire diameter not larger than 0.5 mm. Thus, the core wire has high pushability and is prevented from fatigue deformation, and the core wire can be used suitably for guide wire to be inserted into a narrow blood vessel of a heart, a brain, etc.

**Brief Description of the Drawings**

Fig. 1 is sectional view showing an embodiment of a core wire for guide wire according to the invention.

Figs. 2A to 2C illustrate a method for manufacturing the guide wire according to the invention, Fig. 2A illustrating a state before wire drawing, Fig. 2B illustrating a state where the wire drawing is being performed, Fig. 2C illustrating a state where swaging is being performed.

Fig. 3 is a stress-strain diagram of Comparative Examples 1 to 3 for examining influence of heat treatment on materials subjected to wire drawing.

Fig. 4 is a stress-strain diagram of Comparative Examples 4 to 6 for examining influence of heat treatment on materials subjected to swaging.

Fig. 5 is a stress-strain diagram of Comparative Example 1 and Examples 1 to 3 for examining influence of heat treatment on materials subjected to both wire drawing and swaging.

Fig. 6 is a stress-strain diagram of Comparative Example 7 and Examples 4 and 5 for examining influence of kind of swaging on materials subjected to wire drawing.

Figs. 7A and 7B show an observed texture of Example 1 by a transmission electron microscope, Fig. 7A being a photograph of the texture, Fig. 7B being an electron diffraction pattern.

Figs. 8A and 8B show an observed texture of Example 1 by the transmission electron microscope at a different place from that in Figs. 7A and 7B, Fig. 8A being a photograph of the texture, Fig. 8B being an electron diffraction pattern.

Figs. 9A and 9B show an observed texture of Comparative Example 1 by the transmission electron microscope, Fig. 9A being a photograph of the texture, Fig. 9B being an electron diffraction pattern.

Figs. 10A and 10B show an observed texture of Comparative Example 4 by the transmission electron microscope, Fig. 10A being a photograph of the texture, Fig. 10B being an electron diffraction pattern.

Fig. 11 is a graph showing results of Vickers hardness tests on Comparative Examples 1 and 4 and Example 1.

## Mode for Carrying Out the Invention

An embodiment of a core wire for guide wire according to the invention will be described below with reference to the drawings.

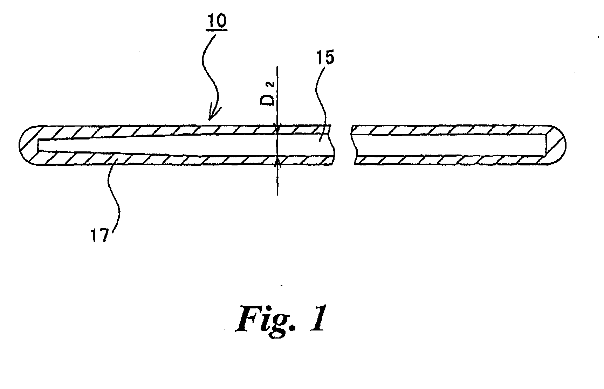

This core wire for guide wire (hereinafter referred to as "core wire") is used so as to be disposed in an internal center of a guide wire. For example, the core wire can be applied to a guide wire 10 shown in Fig. 1. The guide wire 10 in this embodiment includes a core wire 15 according to the invention and a resin layer 17 applied on an outer circumference of the core wire 15. A front end portion of the core wire 15 is tapered off with a diameter gradually reduced toward the tip.

The core wire in this embodiment is made of a Ti-Ni based alloy subjected to wire drawing and swaging. The core wire is formed to have a wire diameter not larger than 0.5 mm and a Young's modulus not lower than 50 GPa.

A Ti-Ni based alloy which is good in biocompatibility and suitable for a guide wire, such as Ti-Ni, Ti-Ni-Cu, Ti-Ni-Fe, Ti-Ni-Nb, etc., is used as the material of the core wire. In addition, alloy elements such as V, Cr, Mn, Co, etc. may be added to the Ti-Ni based alloy (each of the alloy elements has a role of reducing transformation temperature).

The core wire made of the aforementioned Ti-Ni based alloy is further subjected to wire drawing and swaging. In the "wire drawing", a raw material is drawn out through a dice with a certain hole shape or the raw material is inserted into the hole of the dice and extruded therefrom, thereby processing the raw material into a wire with a certain shape. In the "swaging", an outer circumference of the raw material is hammered and drawn using a rotating dice, thereby processing the raw material into a desired shape.

A working ratio $P_1$ when the raw material is drawn is preferably not lower than 10%, more preferably not lower than 20%, and the most preferably not lower than 30%. When the working ratio $P_1$ is lower than 10%, the work hardening of the raw material is insufficient so that the rawmaterial cannot have satisfactory rigidity. The working ratio $P_1$ mentioned herein can be derived from the following Expression (i), in which $A_0$ designates a sectional area of the raw material and $A_1$ designates a sectional area of the raw material (primary processed material) subjected to wire drawing. Although it is preferable that the working ratio is higher, a contrivance for processing may be necessary when the working ratio is higher than 60%.

$$\text{Working Ratio } P_1(\%) = \{(A_0 - A_1)/A_0\} \times 100 \ ... (i)$$

The processing order is not limited as long as the core wire is subjected to both the wire drawing and the swaging, but it is preferable that after wire drawing (primary process) is performed on the raw material, swaging (secondary process) is performed on the raw material (primary processed material) subjected to the wire drawing. In this case, a working ratio $P_2$ when the primary processed material is swaged is preferably not lower than 5%. Further, a total working

ratio $(P_1+P_2)$ of this working ratio $P_2$ and the aforementioned working ratio $P_1$ is preferably not lower than 50%. When the working ratio $P_2$ is lower than 5%, the work hardening of the raw material is insufficient so that the raw material cannot have satisfactory rigidity. The working ratio $P_2$ mentioned herein can be derived from the following Expression (ii), in which $A_1$ designates a sectional area of the primary processed material and $A_2$ designates a sectional area of the raw material (secondary processed material) subjected to swaging.

$$\text{Working Ratio } P_2(\%) = \{(A_1-A_2)/A_1\} \times 100 \ ...(ii)$$

The core wire subjected to the two processes of the wire drawing and the swaging is formed to have a wire diameter not larger than 0.5 mm and a Young's modulus not lower than 50 GPa. As shown in Fig. 1, in the core wire 15 according to this embodiment, any other portion than the tapered front end portion has a wire diameter $D_2$ not larger than 0.5 mm. When the wire diameter of the core wire is larger than 0.5 mm, the core wire can be used for the aorta etc., but it is difficult to use the core wire for coronary intervention (PTCA), a peripheral system, etc. A practical wire diameter applied to PTCA or a peripheral system in an existing guide wire is 0.24 to 0.34 mm.

The core wire having a wire diameter not larger than 0.5 mm is made to have a Young's modulus not lower than 50 GPa. The Young's modulus mentioned herein shows a value when the strain of the core wire is 2%. That is, this core wire has high rigidity to be not lower than 50 GPa in spite of the fine wire diameter not larger than 0.5 mm. When the Young's modulus of the core wire is lower than 50 GPa, the core wire is apt to come short of pushability or torque transmission characteristic which is however required when the core wire is inserted into the microscopic blood vessel such as a coronary artery or a cerebral artery. Thus, the core wire having the Young's modulus lower than 50 GPa is not suitable as a core wire according to the invention. In addition, it is preferable that the Young's modulus is higher.

In this manner, the core wire in this embodiment is subjected to the two processes of wire drawing and swaging, so that the working ratio of the core wire can be improved as compared with the case where the core wire is subjected to the wire drawing alone or the case where the core wire is subjected to the swaging alone. Thus, the core wire can attain high rigidity to be not lower than 50 GPa in Young's modulus in spite of a fine wire diameter not larger than 0.5 mm.

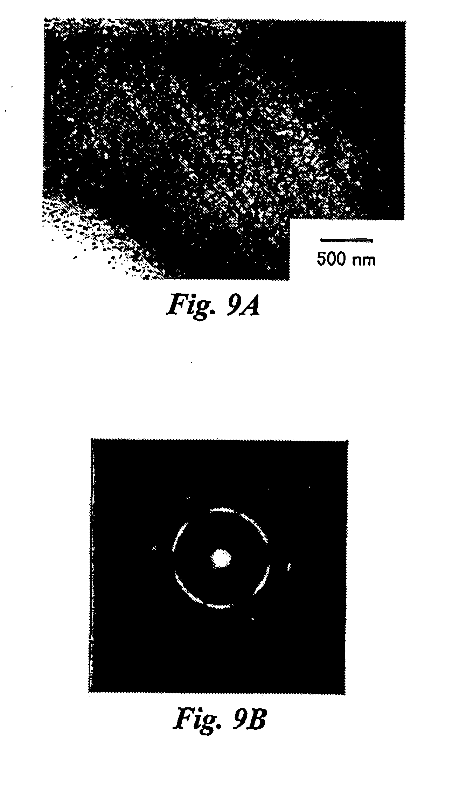

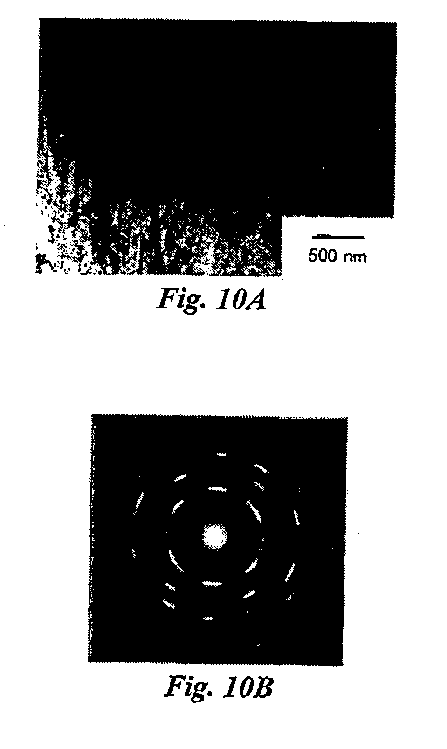

In addition, the texture of this core wire has a microcrystalline structure because the two processes of wire drawing and swaging are performed on the core wire. This will be described with reference to Figs. 9A and 9B (the case where wire drawing is performed alone) and Figs. 10A and 10B (the case where swaging is performed alone). In the case where wire drawing is performed alone as shown in Fig. 9B and the case where swaging is performed alone as shown in Fig. 10B, an image of unclear rings whose outlines are intermittent is observed in an electron diffraction pattern of each core wire. This electron diffraction pattern is an image peculiar to an amorphous structure (so-called halo pattern) In the case where wire drawing is performed alone and the case where swaging is performed alone, the texture of each core wire has an amorphous structure, as is also confirmed with reference to pictures of textures shown in Figs. 9A and 10A.

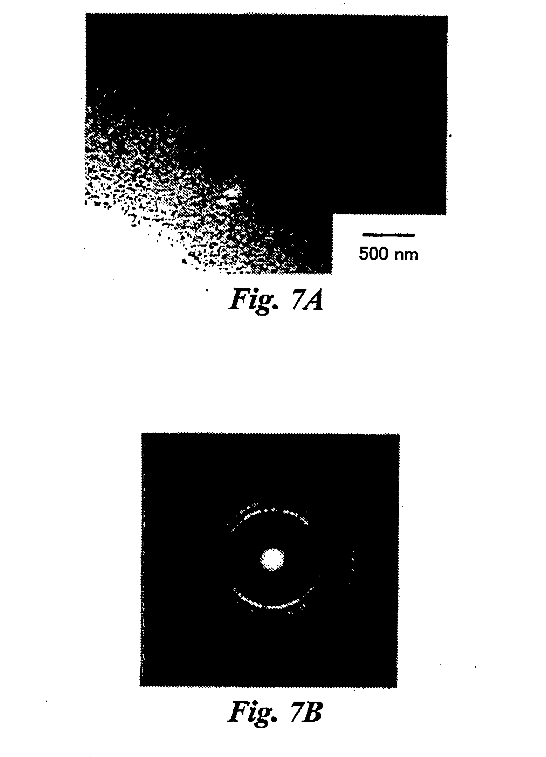

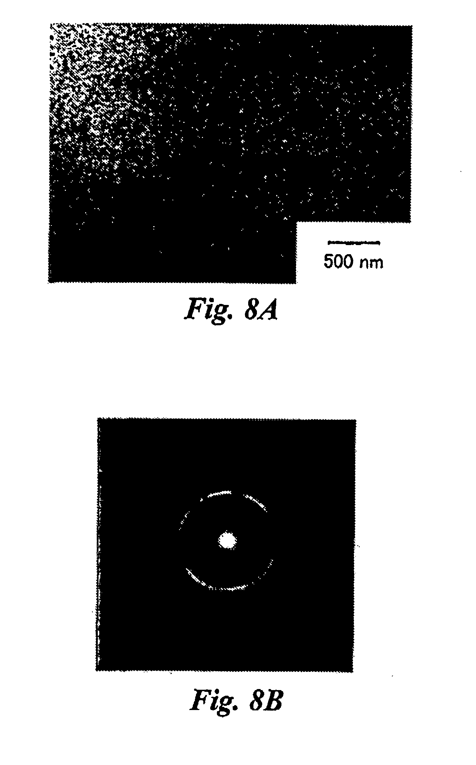

On the other hand, an electron diffraction pattern of a core wire according to the invention is obtained by using and imaging one and the same sample as electron diffraction at two places. As a result, as shown in Figs. 7B and 8B, an image of clear rings can be observed in the electron diffraction pattern. Thus, the core wire has a microcrystalline structure, as is also confirmed clearly with reference to pictures of the textures shown in Figs. 7A and 8A.

In this manner, the textures of the core wire change from a metastable amorphous structure to a stable microcrystalline structure respectively due to both the wire drawing and the swaging performed on the core wire. Thus, the working degree (for example, hardness) increases, and mechanical characteristics such as tensile strength are improved.

The core wire in this embodiment is further subjected to heat treatment so that strain, bending or residual stress caused by processing can be eliminated. For example, the heat treatment can be carried out on the core wire at a temperature of 200 to 400°C for 1 to 60 minutes while the core wire is retained in a certain shape.

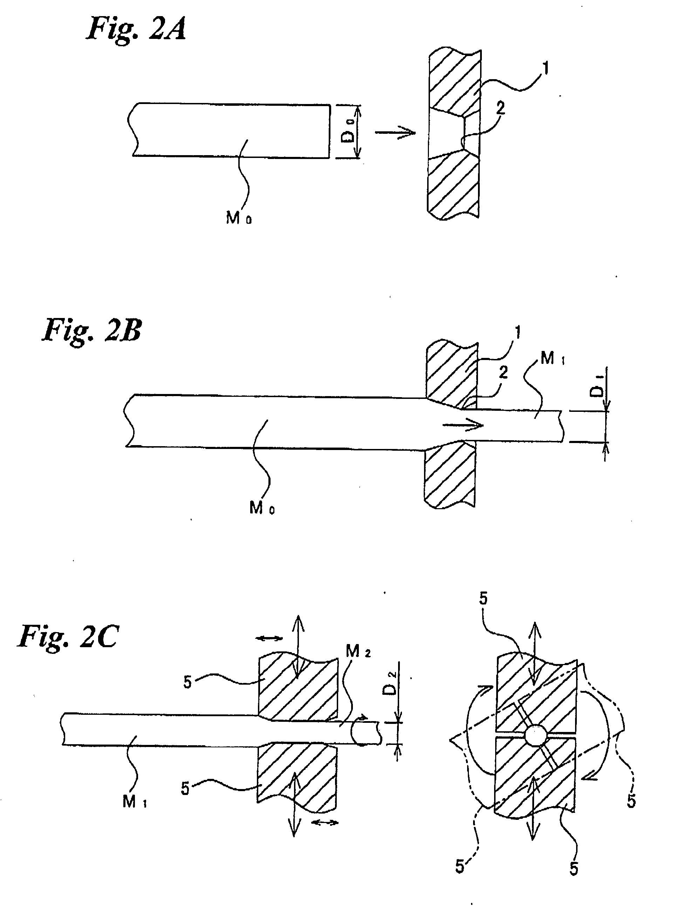

Next, a method for manufacturing a core wire for guide wire according to the invention will be described with reference to Figs. 2A to 2C. The aforementioned core wire for guide wire can be manufactured by this method for manufacturing a core wire for guide wire (hereinafter referred to as "manufacturing method").

This manufacturing method includes a primary process for drawing a raw material $M_0$ into a primary processed material $M_1$ and a secondary process for swaging the primary processed material $M_1$ into a secondary processed material $M_2$ after the primary process, so as to manufacture a core wire which has a wire diameter not larger than 0.5 mm and a Young's modulus not lower than 50 GPa.

First, as shown in Fig. 2A, the raw material $M_0$ having a wire diameter $D_0$ and made of a Ti-Ni based alloy is inserted into a hole 2 formed in a wire-drawing dice 1 and extruded from the hole 2, or the raw material $M_0$ is drawn out through the hole 2 of the wire-drawing dice 1. Thus, as shown in Fig. 2B, wire drawing is performed on the raw material $M_0$ so as to draw the raw material $M_0$ into a certain length while reducing the diameter of the raw material $M_0$. As a result, the primary processed material $M_1$ having a wire diameter $D_1$ is shaped.

The inner diameter of the hole 2 of the wire drawing dice 1 is set so that a working ratio $P_1$ when the raw material $M_0$ is drawn is made preferably not lower than 10%, more preferably not lower than 20%, and most preferably not lower than 30%.

Next, swaging is performed on the primary processed material $M_1$. An apparatus used for the swaging in this embodiment has a pair of swaging dices 5 and 5 as shown in Fig. 2C. The pair of swaging dices 5 and 5 are formed to rotate in certain directions around the primary processed material $M_1$ while colliding with and separating from the primary processed material $M_1$ repeatedly. In addition, the swaging dices 5 and 5 move sliding forward and backward along an axial direction of the primary processed material $M_1$.

Then, the primary processed material $M_1$ is rotated in a certain direction, and the pair of swaging dices 5 and 5 hammer and draw the primary processed material $M_1$ while rotating on the periphery of the primary processed material $M_1$ in a certain direction. At the same time, the same swaging dices 5 move over a certain range in the axial direction of the primary processed material $M_1$. Thus, a secondary processed material $M_2$ having a wire diameter $D_2$ not larger than 0.5 mm is shaped. In this embodiment, an unnecessary portion of the secondary processed material $M_2$ is then further cut off, and a front end portion of the secondary processed material $M_2$ is processed into a tapered shape with a reduced diameter. Thus, the core wire 15 having the wire diameter $D_2$ not larger than 0.5 mm as shown in Fig. 1 is manufactured. The secondary processed material $M_2$ may be used as the core material 15 as it is.

It is also preferable that the size of an arc-like recess formed in a front end portion of each swaging dice is set so that a working ratio $P_2$ when the primary processed material is swaged can be made not lower than 5%. Further, it is preferable that setting is done so that a total working ratio $(P_1+P_2)$ of the working ratio $P_1$ and the working ratio $P_2$ can be made not lower than 50%.

According to this manufacturing method, the two processes of wire drawing and swaging are performed so that the working ratio of the core wire is improved, as compared with the case where wire drawing is performed alone or the case where swaging is performed alone. As a result, the hardness increase caused by work hardening becomes larger. And, it is possible to obtain a core wire which has high rigidity to be not lower than 50 GPa in Young's modulus while having the fine wire diameter not larger than 0.5 mm and which is prevented from fatigue deformation.

Preferably, in this manufacturing method, heat treatment is performed on the core wire after the aforementioned swaging. For example, the heat treatment is performed on the core wire 15 such that the core wire 15 is kept at a temperature 200°C to 400°C for 1 to 60 minutes. Since strain, bending, and residual stress caused by the wire drawing and the swaging are eliminated from the core wire 15 through the heat treatment, it is possible to obtain a core wire which has rigidity and is prevented from fatigue deformation and which has high linearity (not bending but keeping a straight posture in its initial shape).

The core wire according to the invention manufactured by the aforementioned manufacturing method has the following operation and effect.

That is, due to the two processes of wire drawing and swaging performed on the core wire according to the invention, the working ratio of the core wire is improved as compared with the case where wire drawing is performed alone or the case where swaging is performed alone. Thus, the hardness increase caused by work hardening becomes larger than in the case where one of the aforementioned processings is performed alone. Therefore, the core wire according to the invention can attain high rigidity to be not lower than 50 GPa in Young's modulus while having the fine wire diameter not larger than 0.5 mm.

Since the core wire according to the invention has high rigidity while having the fine wire diameter, the core wire is high in pushability which is required when the core wire is inserted into a tubular organ and in toque transmission characteristic which is requested for selection of a blood vessel, and the core wire can be prevented from fatigue deformation. Thus, for example, the core wire according to the invention can be used suitably for guide wire to be inserted into a narrow blood vessel of a heart, a brain, etc.

In the background art, a raw material is drawn and then cut or ground so as to obtain a core wire for guide wire with a narrow size. However, the cost may be increased due to a large waste of the material because the drawn raw material is cut. Further, the hardness and the rigidity may be low because a work-hardened layer produced in the surface of the raw material by the wire drawing is cut.

On the other hand, the wire diameter of the core wire is made not larger than 0.5 mm by the swaging in which the raw material is hammered and drawn. Thus, the yield of the material can be improved to reduce the cost, as compared with the case where the raw material is cut into a certain size. Further, since the swaging is performed after the wire drawing in this embodiment, a work-hardened layer produced by the wire drawing can be prevented from being cut, but the work hardening is further advanced. Thus, a core wire having high rigidity to be not lower than 50 GPa in Young's modulus can be obtained.

**Examples**

Mechanical characteristics, textures and hardness of various core materials were evaluated.

**(Prerequisite) Composition of Ti-Ni. Based Alloy**

Examples and Comparative Examples which will be described below were manufactured using an alloy of Ti-51Ni (at%).

**(1) Manufacturing of Comparative Examples and Examples**

(Comparative Example 1) A linear raw material was cut out from a Ti-Ni based alloy having the aforementioned composition, and wire drawing was performed on the raw material. Thus, Comparative Example 1 was manufactured. The wire diameter was 0.475 mm, and the working ratio rate was 55%.

(Comparative Example 2) A certain length of the raw material was cut out from Comparative Example 1, and heat treatment was applied to the raw material such that the raw material was kept at 200°C for 30 minutes. Thus, Comparative Example 2 was manufactured. The working ratio was the same as that of Comparative Example 1, and the wire diameter was 0.478 mm (The wire diameter varies depending on oxides during the heat treatment. It is also the same in Comparative Example 3 and Examples 2 and 3).

(Comparative Example 3) A certain length of the raw material was cut out from Comparative Example 1, and heat treatment was applied to the raw material such that the raw material was kept at 300°C for 30 minutes. Thus, Comparative Example 3 was manufactured. The working ratio was the same as that of Comparative Example 1, and the wire diameter was 0.482 mm.

(Comparative Example 4) A linear raw material was cut out from a Ti-Ni based alloy having the aforementioned composition, and swaging was performed on the raw material. Thus, Comparative Example 4 was manufactured. The wire diameter was 0.360 mm, and the working ratio was 98.9%.

(Comparative Example 5) A certain length of the raw material was cut out from Comparative Example 4, and heat treatment was applied to the raw material such that the raw material was kept at 200°C for 30 minutes. Thus, Comparative Example 5 was manufactured. The working ratio and the wire diameter were the same as those of Comparative Example 4.

(Comparative Example 6) A certain length of the raw material was cut out from Comparative Example 4, and heat treatment was applied to the raw material such that the raw material was kept at 300°C for 30 minutes. Thus, Comparative Example 6 was manufactured. The working ratio and the wire diameter were the same as those of Comparative Example 4.

(Comparative Example 7) In the same manner as Comparative Example 1, a linear raw material was cut out from a Ti-Ni based alloy having the aforementioned composition, and wire drawing was performed on the raw material. Thus, Comparative Example 7 was manufactured. The wire diameter was 0.602 mm, and the working ratio was 43.8%.

(Example 1) A certain length of the raw material was cut out from Comparative Example 1, and swaging was performed on the raw material. Thus, Example 1 was manufactured. The wire diameter was 0.423 mm and the working ratio after the swaging was 64%.

(Example 2) A certain length of the raw material was cut out from Example 1, and heat treatment was applied to the raw material such that the raw material was kept at 200°C for 30 minutes. Thus, Example 2 was manufactured. The working ratio was the same as that of Example 1 and the wire diameter was 0.416 mm.

(Example 3) A certain length of the raw material was cut out from Example 1, and heat treatment was applied to the raw material such that the raw material was kept at 300°C for 30 minutes. Thus, Example 3 was manufactured. The working ratio was the same as that of Example 1 and the wire diameter was 0.420 mm.

(Example 4) A certain length of the raw material was cut out from Comparative Example 7, and swaging was performed on the raw material with three swaging dices. Thus, Example 4 was manufactured. The wire diameter was 0.486 mm and the working ratio after the swaging was 64%.

(Example 5) A certain length of the raw material was cut out from Comparative Example 7, and swaging was performed on the raw material with two swaging dices. Thus, Example 5 was manufactured. The wire diameter was 0.512 mm and the working ratio after the swaging was 59%.

**(2) Evaluation of Mechanical Characteristics**

**(a) Test Method**

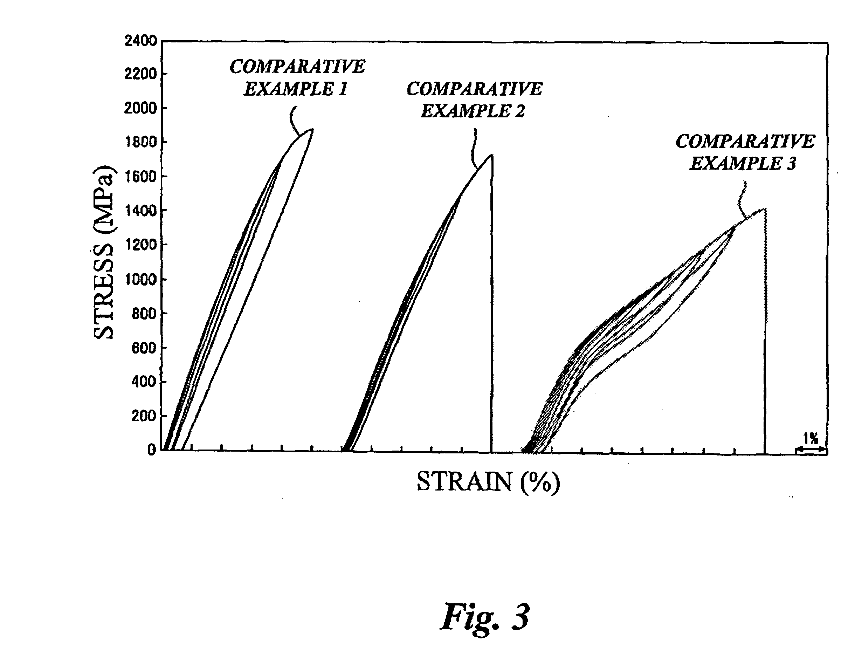

Each of Comparative Examples 1 to 7 and Examples 1 to 5 was processed so that a test piece with a gauge length of 50 mm was manufactured. Each of these test pieces was set in a Tensilon tensile tester to repeat a cycle (load application, load removal, load application, ...) of applying a tensile load to the test piece at a room temperature and with a test speed of 1 mm/min so as to produce an elongation strain in the test piece, and then removing the tensile load from the test piece. The relation between tensile stress (MPa) and strain (%) was measured in each test piece. The results are shown in Figs. 3 to 6 and in Table 1. The Young's modulus on this occasion was calculated from the tensile stress at the strain of 2%.

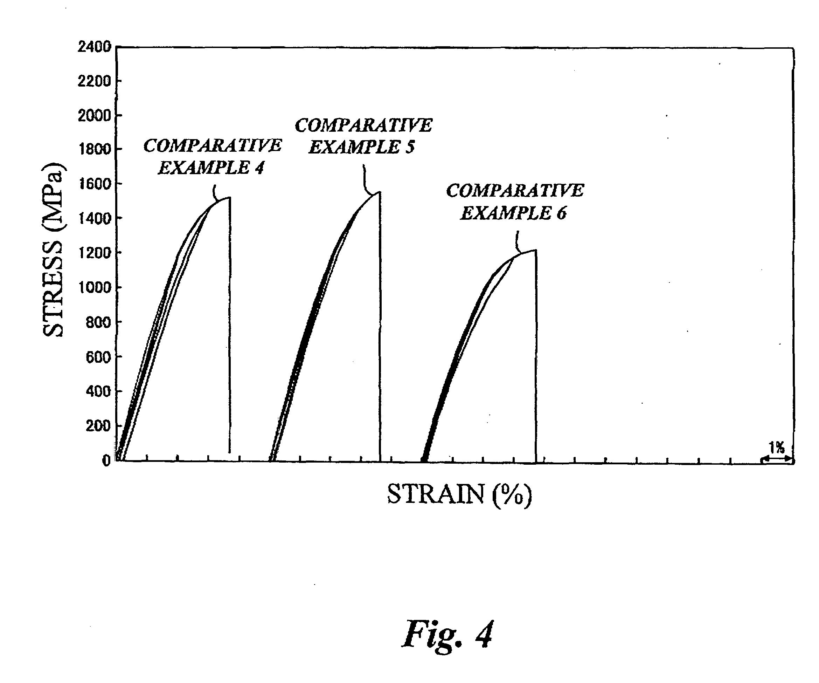

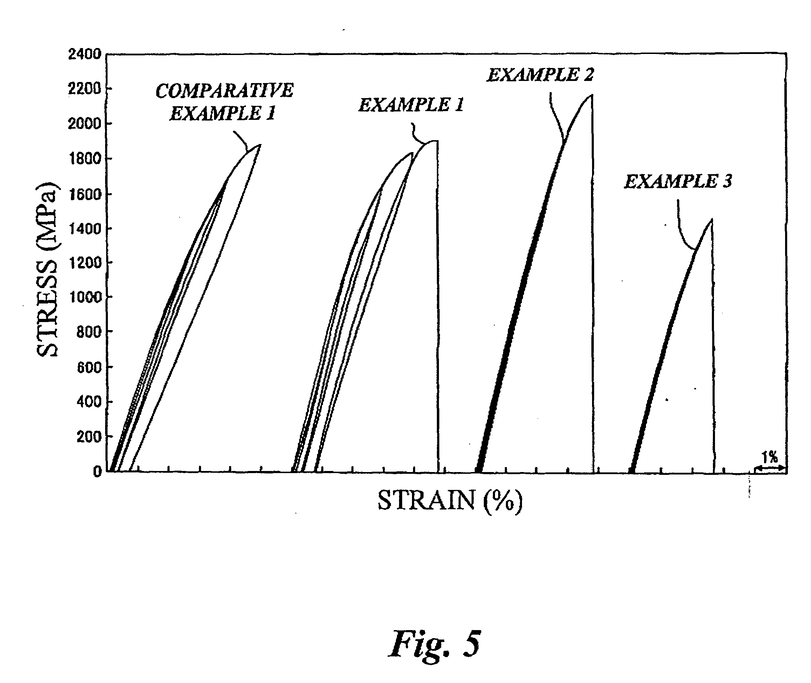

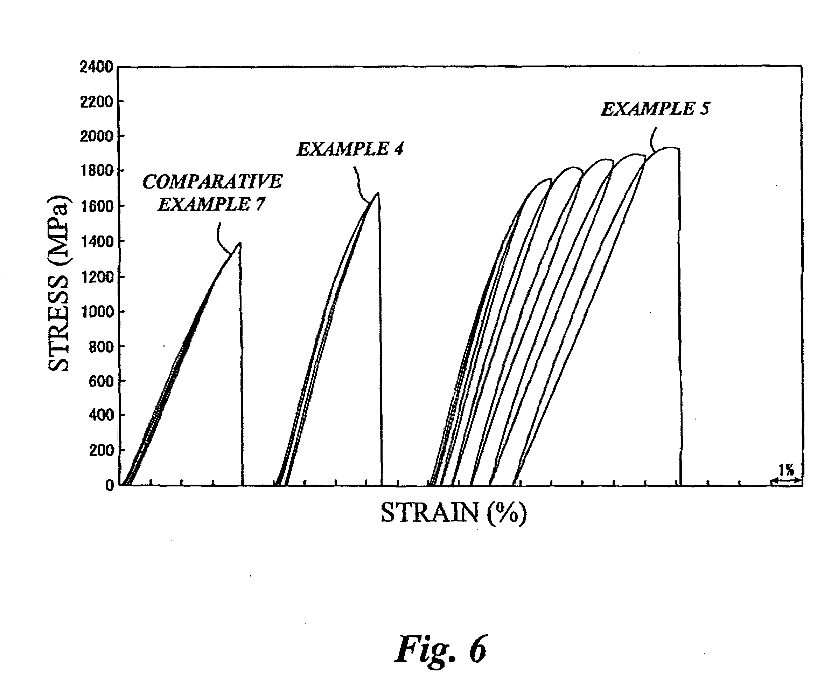

In addition, Comparative Examples 1 to 3 are provided for examining influence of heat treatment on materials subjected to wire drawing (see Fig. 3), and Comparative Examples 4 to 6 are provided for examining influence of heat treatment on materials subjected to swaging (see Fig. 4). On the other hand, Examples 1 to 3 are provided for examining influence of heat treatment on materials subjected to both wire drawing and swaging (see Fig. 5), and Examples 4 and 5 are provided for examining influence of kind of swaging (two dices or three dices) on materials subjected to swaging (see Fig. 6).

[Table 1]

| Target | Drawing | Sample | Wire Drawing Working Ratio (%) | Swaging Working Ratio (%) | Heat Treatment | Wire Diameter (mm) | Young's Modulus (GPa) | Note |
|---|---|---|---|---|---|---|---|---|
| Influence of Heat Treatment on Materials Subjected to Wire Drawing | Fig. 3 | CE1 | 55 | - | - | 0.475 | 49.2 | - |
| | | CE2 | ditto | - | 200°C×30min | 0.478 | 43.8 | heat treatment on sample obtained from CE1 |
| | | CE3 | ditto | - | 300°C×30min | 0.482 | 32.6 | heat treatment on sample obtained from CE1 |
| Influence of Heat Treatment on Materials Subjected to Swaging | Fig. 4 | CE4 | - | 98.9 | - | 0.360 | 58.9 | - |
| | | CE5 | - | ditto | 200°C×30min | ditto | 58.2 | heat treatment on sample obtained from CE4 |
| | | CE6 | - | ditto | 300°C×30min | ditto | 49.6 | heat treatment on sample obtained from CE4 |
| Influence of Heat Treatment on Materials Subjected to Wire Drawing and Swaging | Fig. 5 | Ex1 | 55 | 64 | - | 0.423 | 63.4 | swaging on sample obtained from CE1 |
| | | Ex2 | ditto | ditto | 200°C×30min | 0.418 | 70.2 | heat treatment on sample obtained from Ex1 |
| | | Ex3 | ditto | ditto | 300°C×30min | 0.420 | 60.5 | heat treatment on sample obtained from Ex1 |
| Influence of Kind of Swaging (with Three Dices or Two Dices) on Materials Subjected to Wire Drawing | Fig. 6 | CE7 | 43.8 | - | - | 0.602 | 40.7 | same conditions as CE1 (different sample) |
| | | Ex4 | ditto | 64 | - | 0.466 | 60.9 | 3-dice swaging on sample obtained from CE7 |
| | | Ex5 | ditto | 59 | - | 0.512 | 60.7 | 2-dice swaging on sample obtained from CE7 |

(CE = Comparative Example)

(Ex = Example)

**(b) Discussion of Test Results**

As shown in Fig. 3, it is understood that the Young's modulus is reduced when heat treatment is performed after wire drawing (Comparative Examples 2 and 3). The higher the temperature of the heat treatment is, the more conspicuous that tendency is. The same tendency is confirmed in the case where heat treatment is performed after swaging as shown in Fig. 4.

Fig. 5 shows a stress-strain diagram of Examples 1 to 3. As is understood from the result of Example 1, the material subjected to the two processes of wire drawing and swaging has a high rigidity of 63.4 GPa in Young's modulus in spite of a fine wire diameter of 0.423 mm.

In Comparative Examples shown in Figs. 3 and 4, heat treatment leaded to reduction in Young's modulus. On the other hand, there was obtained a result in Example 2 shown in Fig. 5 that the Young's modulus was improved in spite of heat treatment performed. That is, obtained was the finding that the Young's modulus was improved by heat treatment on the material subjected to the two processes of wire drawing and swaging while the Young's module was reduced by heat treatment on the material subjected to wire drawing or swaging only. This is because the metal texture has a microcrystalline structure due to the combination of wire drawing and swaging. That is, heat treatment performed after wire drawing or heat treatment performed after swaging leads to loss in energy (reduction in Young's modulus) due to recrystallization, while heat treatment performed after wire drawing and swaging leads to no loss in energy (no reduction in Young's modulus).

Fig. 6 shows Young's modulus in the case where swaging was performed with three dices (Example 4) and Young's modulus in the case where swaging was performed with two dices (Example 5). The number of dices had little influence, but it could be confirmed that the Young's modulus was improved in each case.

### (3) Evaluation of Textures

Textures and electron diffraction patterns of Comparative Examples 1 and 4 and Example 1 were observed with a transmission electron microscope (TEM). The results are shown in Figs. 7A to 8B (Example 1), Figs. 9A and 9B (Comparative Example 1) and Figs. 10A and 10B (Comparative Example 4). Incidentally, Example 1 was observed in two different positions (Figs. 7A to 8B).

As shown in Figs. 9B and 10B, the electron diffraction patterns of Comparative Examples 1 and 4 have unclear images peculiar to an amorphous structure. Also from Figs. 9A and 10A, it is understood that structures in Comparative Examples 1 and 4 are amorphous.

On the other hand, as shown in Figs. 7B and 8B, the electron diffraction pattern of Example 1 has a clear ring-like image peculiar to a crystalline structure. Further as shown in Figs. 7A and 8B, it is also understood from the texture pictures that the texture of Example 1 has a microcrystalline structure. Thus, the two processes of wire drawing and swaging lead to a microcrystalline structure in the texture.

### (4) Evaluation of Hardness

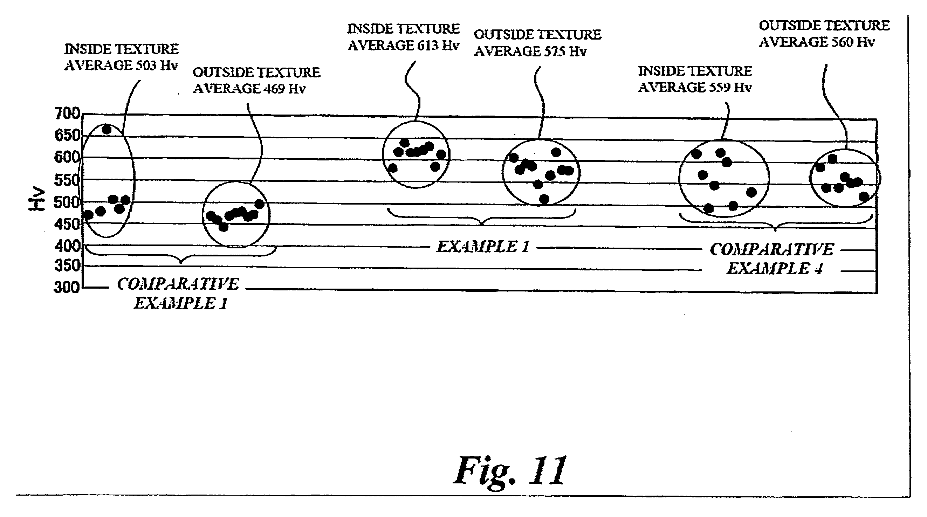

Vickers hardness (Hv) was measured in Comparative Examples 1 and 4 and Example 1 whose textures were observed in (3). Vickers hardness was measured outside the texture and inside the texture in each sample with a load of 200 kg. The results are shown in Fig. 11.

As shown in Fig. 11, the Vickers hardness in Comparative Example 1 subjected to wire drawing only is comparatively low, and the Vickers hardness in Comparative Example 4 subjected to swaging only is comparatively high but varies widely. On the other hand, in Example 1 subjected to wire drawing and swaging, the Vickers hardness is high both inside the texture and outside the texture, and low in variation. It is therefore understood that the texture structure of Example 1 is highly stable.

### Description of Preference Numerals and Signs

10    guide wire
15    core wire for guide wire (core wire)

### Claims

1.  A core wire for guide wire,
    wherein the core wire (15)
    is made of a Ti-Ni based alloy,
    has a wire diameter ($D_2$) not larger than 0.5 mm and **characterized in that** the core wire has a Young's modulus not lower than 50 Gpa,
    and
    **in that** the core wire (15) is produced by wire drawing and subsequent swaging.

2. The core wire of Claim 1, wherein the Ti-Ni based alloy is a work-hardened Ti-Ni based alloy whose texture has a microcrystalline structure.

3. The core wire of Claim 1,
wherein heat treatment as well as the wire drawing and the swaging is performed on the core wire (15).

4. A method for manufacturing the core wire of Claims 1, the method comprising:

drawing a raw material ($M_0$) made of a Ti-Ni based alloy, and then carrying out swaging to obtain a core wire (15) having a wire diameter ($D_2$) not larger than 0.5 mm and a Young's modulus not lower than 50 GPa.

5. The method of Claim 4, further comprising:

performing heat treatment after the swaging.

**Patentansprüche**

1. Kerndraht für einen Führungsdraht,
wobei der Kerndraht (15)
aus einer Ti-Ni-basierten Legierung gefertigt ist,
einen Drahtdurchmesser ($D_2$) von nicht mehr als 0,5 mm hat und **dadurch gekennzeichnet ist, dass** der Kerndraht einen E-Modul von nicht weniger als 50 GPa hat,
und dadurch, dass
der Kerndraht (15) durch Drahtziehen und anschließendes Gesenkschmieden hergestellt wird.

2. Kerndraht nach Anspruch 1, bei dem die Ti-Ni-basierte Legierung eine kaltverfestigte Ti-Ni-basierte Legierung ist, deren Textur eine mikrokristalline Struktur hat.

3. Kerndraht nach Anspruch 1,
bei dem die Wärmebehandlung wie auch das Drahtziehen und das Gesenkschmieden an dem Kerndraht (15) ausgeführt werden.

4. Verfahren zum Herstellen des Kerndrahtes nach Anspruch 1, wobei das Verfahren umfasst:

Ziehen eines Rohmaterials ($M_0$), das aus einer Ti-Ni-basierten Legierung besteht, und anschließendes Ausführen des Gesenkschmiedens, um einen Kerndraht (15) zu erhalten, der einen Drahtdurchmesser ($D_2$) von nicht mehr als 0,5 mm und einen E-Modul von nicht weniger als 50 GPa hat.

5. Verfahren nach Anspruch 4, weiterhin umfassend:

Ausführen einer Wärmebehandlung nach dem Gesenkschmieden.

**Revendications**

1. Fil d'âme pour fil de guidage,
où le fil d'âme (15)
est fait d'un alliage à base de Ti-Ni,
a un diamètre de fil ($D_2$) non supérieur à 0,5 mm et **caractérisé en ce que** le fil d'âme a un module d'Young non inférieur à 50 Gpa,
et **en ce que**
le fil d'âme (15) est produit par étirage de fil et estampage subséquent.

2. Fil d'âme selon la revendication 1, où l'alliage à base de Ti-Ni est un alliage à base de Ti-Ni durci à froid dont la texture a une structure microcristalline.

3. Fil d'âme selon la revendication 1,

où un traitement thermique ainsi que l'étirage de fil et l'estampage sont mis en oeuvre sur le fil d'âme (15).

4. Procédé pour fabriquer le fil d'âme selon la revendication 1, le procédé comprenant :

l'étirage d'une matière première ($M_o$) faite d'un alliage à base de Ti-Ni, puis la conduite d'un estampage pour obtenir un fil d'âme (15) ayant un diamètre de fil ($D_2$) non supérieur à 0,5 mm et un module d'Young non inférieur à 50 GPa.

5. Procédé selon la revendication 4, comprenant en outre :

la mise en oeuvre d'un traitement thermique après l'estampage.

## *Fig. 1*

## Fig. 2A

## Fig. 2B

## Fig. 2C

*Fig. 3*

Fig. 4

*Fig. 5*

Fig. 6

*Fig. 7A*

*Fig. 7B*

500 nm

*Fig. 8A*

*Fig. 8B*

*Fig. 9A*

*Fig. 9B*

**Fig. 10A**

**Fig. 10B**

*Fig. 11*

**EP 2 404 636 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H05293175 A
- JP H06165822 A
- JP H11128363 A
- WO 0141859 A